# EUROPÄISCHE PATENTSCHRIFT

(11) **EP 0 521 314 B1**
(45) Veröffentlichungstag und Bekanntmachung des Hinweises auf die Patenterteilung: **12.04.1995**
(21) Anmeldenummer: 92109622.8
(22) Anmeldetag: 06.06.1992
(51) Int. Cl.: A61M 16/00

(54) **Verfahren zur Erkennung der Atemphasen eines Patienten bei assistierenden Beatmungsverfahren**
Method for detecting the breathing phases of a patient under assisted ventilation
Méthode de détection des phases respiratoires d'un patient sous respiration assistée

(30) Priorität: 04.07.1991 DE 4122069
(43) Veröffentlichungstag der Anmeldung: 07.01.1993
(73) Patentinhaber: Drägerwerk Aktiengesellschaft, 23542 Lübeck (DE)
(72) Erfinder: Weismann, Dieter, Dr., W-2401 Gross Grönau (DE); Baum, Marcel, A-6405 Oberhofen-Telfs (DE)

(56) Entgegenhaltungen:
- EP-A- 0 046 570
- US-A- 3 834 382
- US-A- 4 050 458

## Beschreibung

Die Erfindung betrifft ein Verfahren zur Erkennung der Atemphasen eines Patienten zur Steuerung eines Beatmungsgerätes, wobei der zeitliche Verlauf eines die Atemströmungskurve repräsentierenden, von einer Signalvorrichtung abgegebenen Signals ermittelt wird und aus diesem Verlauf ein Triggerkriterium für die Umsteuerung des Beatmungsgerätes von der Exspirationsphase in die Inspirationsphase und/ oder von der Inspirationsphase in die Exspirationsphase gewonnen wird

Beim lungengesunden Patienten besteht zwischen Kraftverlauf der Atemmuskulatur und der resultierenden Atemströmung keine nennenswerte Phasenverschiebung. Deshalb kann aus den Nulldurchgängen von Munddruck oder Atemströmung eine gute Atemphasenerkennung abgeleitet werden, wie sie für die Phasenumsteuerung bei assistierenden Beatmungsformen als Triggerkriterium benötigt wird.

In der US-PS-3,834,382, von welcher der Oberbegriff des Anspruchs 1 ausgeht, ist ein Beatmungsgerät beschrieben, bei dem der Nulldurchgang der Atemströmungskurve als Triggerkriterium benutzt wird. Dieses Verfahren versagt aber, wenn die Zeitkonstante der Lunge in bezug auf die Dauer der Atemphasen zunimmt, wie dies bei chronisch obstruktiven Patienten (COPD) bei erhöhter Atemfrequenz der Fall ist. Es entsteht eine Phasenverschiebung zwischen dem Kraftverlauf der Atemmuskulatur und der resultierenden Atemströmung. Man beobachtet dann eine Diskoordination zwischen der Aktivität der Atemmuskulatur und der Strömungsrichtung in den Atemwegen und somit den äußerlich erkennbaren Atemphasen.

Ein assistierendes Beatmungsverfahren, welches die Umsteuerung von Inspiration auf Exspiration bzw. von Exspiration auf Inspiration nach den heute verwendeten Methoden der Atemphasenerkennung vornimmt, kann bei solchen Patienten zu einer unerwünschten Erhöhung der isometrischen Atemarbeit beitragen. Der Patient empfindet den Einsatz der Inspirationshilfe als zu spät, andererseits leitet er bereits eine Exspiration ein, obwohl das Beatmungsgerät noch immer Volumen in die Lunge liefert.

In der US-PS 4,050,458 ist ein Beatmungsgerät beschrieben, bei dem der zeitliche Verlauf des Munddruckes des Patienten gemessen und durch Bilden des zweiten zeitlichen Differentials ein Knickpunkt in der Endphase des exspiratorischen Druckverlaufs bestimmt wird. Dieser Knickpunkt dient als Triggerkriterium für die nachfolgende Inspirationsphase. Nachteilig an diesem Gerät ist, daß ein sicher auswertbares Drucksignal nur zu erhalten ist, wenn das Beatmungsgerät einen unerwünscht hohen Strömungswiderstand aufweist. Bei üblichen Strömungswiderständen ist das Drucksignal so klein, daß es im Rauschen untergeht. Weiterhin ist es von Nachteil, daß kein Triggerkriterium für die Umschaltung von Inspiration auf Exspiration gewonnen werden kann.

Der Erfindung liegt daher die Aufgabe zugrunde, ein Verfahren zur Erkennung der Atemphasen eines Patienten zur Steuerung eines Beatmungsgerätes, wobei der zeitliche Verlauf eines die Atemströmungskurve repräsentierenden, von einer Signalvorrichtung abgegebenen Signals ermittelt wird und aus diesem Verlauf ein Triggerkriterium für die Umsteuerung des Beatmungsgerätes von der Exspirationsphase in die Inspirationsphase und/ oder von der Inspirationsphase in die Exspirationsphase gewonnen wird, anzugeben, das auch bei chronisch obstruktiven Patienten ein sicheres und frühzeitiges Erkennen des Ein- und Ausatemversuchs ermöglicht.

Die Aufgabe wird dadurch gelöst, daß als Triggerkriterium eine signifikante Zunahme der Steilheit der Atemströmungskurve jeweils zwischen ihren Nulldurchgängen benutzt wird.

Der Vorteil der Erfindung liegt darin, daß durch das Verfahren jeder Versuch des Patienten, ein- oder auszuatmen, auch bei Vorliegen einer Phasenverschiebung zwischen dem Kraftverlauf der Atemmuskulatur und der resultierenden Atemströmung erkannt wird. Durch die Verwendung eines die Atemströmung repräsentierenden Signals ist eine sichere Auswertung der Meßwerte möglich.

Die Erfindung mit ihren in den Unteransprüchen beschriebenen Ausgestaltungen wird im folgenden beschrieben und noch einmal den bekannten Verfahren gegenübergestellt.

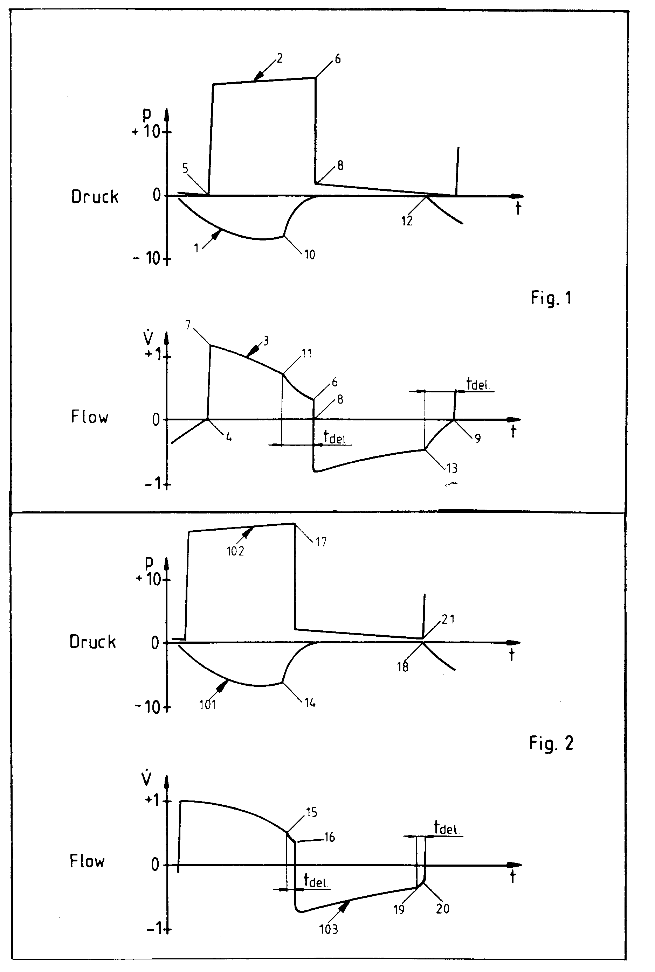

In der Zeichnung sind in Fig. 1 in willkürlichen Einheiten Druck- und Strömungsmeßwerte, wie sie sich bei einer Beatmung eines chronisch obstruktiven Patienten nach bekannten Verfahren ergeben, über der Zeit dargestellt; Fig. 2 zeigt im Vergleich dazu die entsprechenden Kurvenverläufe, die sich bei dem erfinderischen Verfahren ergeben.

In Fig. 1 sind im oberen Teil der Pleuraldruck (1) und der Atemwegsdruck (2) dargestellt. Der Pleuraldruck ist der Druck zwischen Lunge und Brustkorb des Patienten und stellt ein Maß für die Aktivität der Atemmuskulatur dar. Der Atemwegsdruck ist der Druck des Atemgases am Mund des Patienten.

Im unteren Teil von Fig. 1 ist die Atemströmung (3) dargestellt.

Die Kurven beginnen mit der Endphase einer Exspiration. Der Nulldurchgang (4) der Atemströmung (3) dient dem nach dem bekannten Verfahren arbeitenden Beatmungsgerät als Triggerkriterium für die Umschaltung auf Inspiration. In dem Moment (5) steigt der von dem Beatmungsgerät erzeugte Atemwegsdruck (2) und folglich auch die Atemströmung (3) plötzlich an. Der Atemwegsdruck (2) steigt bis zum Ende (6) der Inspirationsphase leicht an, während die Atemwegströmung (3) ständig abnimmt. Als Triggerkriterium für die Umschaltung auf Exspiration dient dem nach dem bekannten Verfahren arbeitenden Beatmungsgerät der Moment (6), bei dem die Atemströmung auf 25 % ihres Maximalwertes (7) abgefallen ist.

Der Atemwegsdruck (2) fällt dann plötzlich auf einen sehr geringen Wert (8), der sich aus der Atemströmung und dem Strömungswiderstand des Beatmungsgerätes ergibt, die Atemströmung (3) wird negativ. Der Atemwegsdruck (2) und der Absolutbetrag der Atemströmung (3) nehmen dann ständig ab, bis beim erneuten Nulldurchgang (9) der Atemströmung durch das Beatmungsgerät eine neue Inspirationsphase eingeleitet wird.

Betrachtet man jetzt den Pleuraldruck (1), so erkennt man, daß im ersten Teil der Inspirationsphase der Pleuraldruck ständig negativer wird. Das bedeutet, daß der Patient seine Atemmuskulatur anspannt, um einzuatmen.

An einem Punkt (10) knickt die Kurve plötzlich ab, der Pleuraldruck geht langsam gegen Null. Der Knickpunkt (10) zeigt an, daß der Patient seine Atemmuskulatur entspannt, er möchte ausatmen.
Bedingt durch die Verengung seiner Atemwege fällt die Atemströmung (3) aber noch nicht stark ab. Es zeigt sich lediglich eine Änderung der Steigung (11) in der Atemströmung (3) zu dem Zeitpunkt (10), an dem die Pleuraldruckkurve (1) abknickt. Das Beatmungsgerät reagiert aber erst dann, wenn die Atemströmung auf 25 % des Maximalwertes abgefallen ist (6), schaltet also viel später als der Patient es möchte auf Exspiration um. In Fig. 1 ist die Zeit vom Versuch des Patienten auszuatmen bis zur Umschaltung des Beatmungsgerätes mit t del gekennzeichnet.

Der Abfall (12) der Pleuraldruckkurve (1) zeigt den Versuch des Patienten an, einzuatmen. In der Atemströmungskurve (3) zeigt sich wieder eine Änderung der Steigung (13). Das Beatmungsgerät reagiert aber erst viel später, wenn nämlich die Atemströmung (3) durch Null (9) geht.

Bei einem Versuch des Patienten einzuatmen, ändert sich die Steigung der Atemströmungskurve (3) grundsätzlich dahingehend, daß sie steiler als vorher auf die Nullinie zuläuft (Punkte 11 und 13 in Kurve 3), die Stei lheit der Atemströmungskurve nimmt also zu.

In Fig. 2 sind die entsprechenden Kurven des Pleuraldruckes (101), des Atemwegsdruckes (102) und der Atemströmung (103), die sich bei einem nach dem erfinderischen Verfahren arbeitenden Beatmungsgerät ergeben, aufgetragen.

In dem Moment, wo der Patient ausatmen möchte, gekennzeichnet durch den Knick (14) in der Kurve des Pleuraldruckes (101), ändert sich die Steigung der Atemströmungskurve (103) (Punkt 15). Das Beatmungsgerät erkennt diese Steigungsänderung schon sehr kurze Zeit später und leitet die Exspirationsphase ein (Punkt 16). Der Atemwegsdruck (102) fällt (Punkt 17) und die Atemströmung (103) wird negativ.

Der fallende Pleuraldruck (Punkt 18) zeigt an, daß der Patient einatmen möchte. Im gleichen Moment (Punkt 19) ändert sich die Steigung der Atemströmungskurve (103) und kurze Zeit später (Punkt 20) schaltet das Beatmungsgerät auf Inspiration um. Der Atemwegsdruck (102) steigt (Punkt 21), die Atemströmung (103) wird positiv.

Die Zeit, die zwischen dem Versuch des Patienten, ein- bzw. auszuatmen, und der Reaktion des Beatmungsgerätes liegt, ist bei dem erfinderischen Verfahren (t del in Fig. 2) erheblich geringer als bei dem bekannten Verfahren (t del in Fig. 1). Dadurch wird der Patient wesentlich schonender beatmet.

Die auszuwertende Atemströmungskurve (3, 103) kann auf unterschiedliche weise gewonnen werden. Zum Beispiel kann ein Flowsensor, der in verschiedenen Bauarten bekannt ist, in die Atemgasleitung des Beatmungsgerätes eingebaut werden, und das elektrische Ausgangssignal dieses Flowsensors repräsentiert dann die Atemströmungskurve. Auf einen Flowsensor kann verzichtet werden bei Beatmungsgeräten, in denen ein Flowregelventil z.B. so angesteuert wird, daß der Atemwegsdruck (Kurve 2 bzw. 102) stets auf einer Sollkurve gehalten wird. Das Steuersignal des Flowregelventils repräsentiert dann die Atemströmungskurve.

Als Triggerkriterium für die Umschaltung des Beatmungsgerätes dient erfindungsgemäß eine Zunahme der Steilheit der Atemströmungskurve. Der Umschaltmoment, an dem die Atemströmungskurve durch Null geht, stellt die stärkste auftretende Zunahme der Steilheit dar, soll aber selbstverständlich nicht als Triggerkriterium dienen, da anderenfalls das Beatmungsgerät ständig mit hoher Frequenz die Atemphasen umschalten würde. Der Zeitpunkt für das Erkennen eines Triggerkriteriums muß daher auf ein Zeitintervall jeweils zwischen den Nulldurchgängen der Atemströmungskurve eingeschränkt werden.

Weiterhin kommt es in einer kurzen Zeitspanne nach einer Umschaltung der Atemphase durch Einschwingvorgänge des Beatmungsgerätes zu Schwankungen in der Atemströmungskurve, die ungewollt als Triggerkriterium erkannt werden könnten. Das Umschalten in die jeweils andere Atemphase ist generell erst sinnvoll, nachdem seit dem letzten Umschalten eine Zeit verstrichen ist, die sich aus dem Kehrwert der doppelten maximal zulässigen Atemfrequenz ergibt.

Aus den vorgenannten Gründen ist es sinnvoll, ein Zusatzkriterium für die Gültigkeit des Triggerkriteriums zu schaffen und so unerwünschte verfrühte Triggerungen auszuschließen. Dies kann dadurch geschehen, daß nur eine, nach Überschreiten des jeweiligen Extremwertes der Atemströmungskurve auftretende signifikante Zunahme der Steilheit der Atemströmungskurve als Triggerkriterium benutzt wird.

Noch mehr Sicherheit gegen unerwünschte Triggerungen erreicht man, wenn nur eine, nach Verstreichen einer mit jedem Nulldurchgang der Atemströmungskurve beginnenden Verzögerungszeit auftretende signifikante Zunahme der Steilheit der Atemströmungskurve als Triggerkriterium benutzt wird.

Die Verzögerungszeit muß mindestens so lang sein, daß die erwähnten Einschwingvorgänge so weit abgeklungen sind, daß sie die Erkennung des Triggerkriteriums nicht mehr stören. Diese Zeit ist abhängig von den Eigenschaften des Beatmungsgerätes, als untere Grenze ist ein wert von ca. 50 ms anzunehmen. Die maximale Verzögerungszeit muß kürzer sein als die Zeit, die sich aus dem Kehrwert der doppelten minimal zulässigen Atemfrequenz ergibt. Bei einer Atemfrequenz von 30 pro Minute ergibt sich eine Sekunde. Als Obergrenze für die Verzögerungszeit kann man unter diesen Bedingungen einen wert von 800 ms ansetzen. Die beschriebene Verknüpfung des Triggerkriteriums mit einem Zusatzkriterium (Überschreiten des Extremwertes bzw. Verzögerungszeit) kann mit bekannten Mitteln wie z.B. logischen elektronischen Schaltungen oder in einem Rechner mittels geeigneter Programmbefehle durchgeführt werden.

Als signifikant für die Erkennung als Triggerkriterium ist eine Zunahme der Steilheit der Atemströmungskurve dann anzusehen, wenn sie innerhalb eines bestimmten Zeitintervalls stattfindet (zwischen den Nulldurchgängen oder nach Überschreiten des Extremwertes der Atemströmungskurve oder nach Ablauf einer Verzögerungszeit) und wenn sie ein festlegbares Maß überschreitet. Dieses Maß wird so festgelegt, daß nicht zufällige Signalschwankungen (Rauschen) oder ein nichtlinearer Verlauf der Atemströmungskurve ungewollt eine Triggerung auslösen können. Stark nichtlinear wird die Atemströmungskurve z.B. wenn das Beatmungsgerät einen während der Inspirationsphase variablen Atemwegsdruck liefert. Durch entsprechende elektronische Mittel ist es möglich, die durch den variablen Atemwegsdruck hervorgerufene Änderung der Steilheit der Atemströmungskurve zu bestimmen und von der gemessenen Änderung der Steilheit der Atemströmungskurve zu subtrahieren. Die Differenz stellt dann hauptsächlich die von einem Ein- oder Ausatemversuch des Patienten herrührende signifikante Änderung der Steilheit der Atemströmungskurve dar.

Die als Triggerkriterium für die Umschaltung der Atemphasen dienende Zunahme der Steilheit der Atemströmungskurve kann auf unterschiedliche Weisen, wie in den Unteransprüchen beschrieben, erkannt werden.

Eine Möglichkeit besteht darin, das die Atemströmungskurve (3, 103) repräsentierende Signal zuerst durch einen Tiefpaß zu glätten, um störendes Signalrauschen zu reduzieren. Dann wird das zweite zeitliche Differential gebildet. Das so gewonnene Signal ist ein Maß für die Krümmung der Atemströmungskurve. In dem Beispiel von Fig. 1, Kurve 3 ist dieses Signal von Punkt 7 bis Punkt 11 leicht positiv und wird am Punkt 11 deutlich negativ. Durch eine Schwellenwertschaltung kann diese signifikante Änderung des Signals von kleinen, durch verbliebenes Rauschen und Nichtlinearität der Atemströmungskurve entstehendne Signalschwankungen unterschieden werden. Das hinter der Schwellenwertschaltung anstehende Signal zeigt dann eine signifikante Änderung in der Steilheit der Atemströmungskurve an und kann einer Triggerschaltung des Beatmungsgerätes zugeführt werden. Diese Triggerschaltung schaltet dann das Beatmungsgerät in die jeweils andere Atemphase um. In diesem Moment ändert sich die Steigung der Atemströmung stark (Punkte 16 und 20 in Kurve 103, Fig. 2), was wiederum zu einem Signal am Ausgang der Schwellwertschaltung führt. Dieses Signal soll aber nicht als Triggerkriterium dienen und kann durch eine logische Schaltung unterdrückt werden.

Eine weitere Möglichkeit, das Triggerkriterium zu finden, besteht darin, eine mathematische Funktion an die Atemströmungskurve jeweils in der Zeit zwischen den Nulldurchgängen anzupassen. Als mathematische Funktion kann ein Polynom n-ter Ordnung oder eine Exponentialfunktion verwandt werden. Der einfachste Fall ist die Anpassung einer Geraden. Dazu wird der Verlauf der eine bestimmte Zeit vor der aktuellen Zeit liegenden Atemströmungswerte linear in die Zukunft extrapoliert.

Ab den Knickpunkten (15, 19) der Atemströmungskurve (103) weichen die gemessenen Werte (103) zunehmend von den mit der angepaßten Funktion berechneten Werten ab. Um eine als Triggerkriterium nutzbare Abweichung durch die Atemtätigkeit des Patienten von einer z. B. durch Signalrauschen hervorgerufenen Abweichung zu unterscheiden, muß die Abweichung ein vorgebbares Maß überschreiten, um als signifikant angesehen zu werden. Ist dieses Maß überschritten (Punkte 16 und 20, Fig. 2), wird ein Signal an die Triggerschaltung des Beatmungsgerätes gegeben.

Die Anpassung der Funktionen für Inspiration und Exspiration kann auch über mehrere Atemzüge gemittelt vorgenommen werden. Aus den Funktionen ergeben sich dann jeweils gemittelte Parameter für den Abfall des Absolutbetrages der Atemströmung in Inspiration und Exspiration. Mit diesen Parametern und den jeweils gemessenen Extremwerten der Atemströmungskurve kann dann der voraussichtliche Verlauf der Atemströmungskurve vorausberechnet werden. Eine signifikante Abweichung des gemessenen Verlaufs vom vorausberechneten dient dann als Triggerkriterium.

In einer letzten Ausführungsform der Erfindung wird auf einem Rechner ein mathematisches Modell der Patientenlunge und des Beatmungsgerätes installiert, das synchron zur Beatmung mitläuft. Die Modellparameter (z.B. Compliance und Resistance der Lunge sowie der vom Beatmungsgerät vorgegebene Atemwegsdruck) werden ständig an die gemessenen Werte der Atemströmungskurve angepaßt. In diesem Rechnermodell treten dann die Zunahmen der Steilheit der Atemströmungskurve (Punkte 15 und 19, Fig. 2) auch auf, werden von dem Rechnerprogramm erkannt und dienen als Triggerkriterium für die Umschaltung des Beatmungsgerätes. Alternativ dazu kann auch wieder eine Abweichung der gemessenen von der berechneten Atemströmungskurve als Triggerkriterium dienen.

Die genannten Ausführungsformen des erfinderischen Verfahrens lassen sich mittels analoger Elektronik (z.B. Analogrechner) realisieren oder nach Digitalisierung der Meßwerte in einem Rechner ausführen.

## Patentansprüche

1. Verfahren zur Erkennung der Atemphasen eines Patienten zur Steuerung eines Beatmungsgerätes, wobei der zeitliche Verlauf eines die Atemströmungskurve repräsentierenden, von einer Signalvorrichtung abgegebenen Signals ermittelt wird und aus diesem Verlauf ein Triggerkriterium für die Umsteuerung des Beatmungsgerätes von der Exspirationsphase in die Inspirationsphase und/oder von der Inspirationsphase in die Exspirationsphase gewonnen wird, dadurch gekennzeichnet, daß als Triggerkriterium eine signifikante Zunahme der Steilheit der Atemströmungskurve jeweils zwischen ihren Nulldurchgängen benutzt wird.

2. Verfahren nach Anspruch 1, dadurch gekennzeichnet, daß nur diejenige, nach Überschreiten des jeweiligen Extremwertes der Atemströmungskurve auftretende signifikante Zunahme der Steilheit der Atemströmungskurve jeweils zwischen ihren Nulldurchgängen als Triggerkriterium benutzt wird.

3. Verfahren nach Anspruch 1 oder 2, dadurch gekennzeichnet, daß nur diejenige nach Verstreichen einer, mit jedem Nulldurchgang der Atemströmungskurve beginnenden Verzögerungszeit auftretende signifikante Zunahme der Steilheit der Atemströmungskurve als Triggerkriterium benutzt wird.

4. Verfahren nach Anspruch 3, dadurch gekennzeichnet, daß für die Verzögerungszeit ein Wert zwischen 50 ms und 800 ms gewählt wird.

5. Verfahren nach einem der Ansprüch 1 bis 4, dadurch gekennzeichnet, daß das die Atemströmungskurve repräsentierende Signal Tiefpaß-gefiltert und danach das zweifache zeitliche Differential gebildet wird und eine eine signifikante Zunahme der Steilheit der Atemströmungskurve repräsentierende Änderung des so gewonnenen Signals jeweils zwischen den Nulldurchgängen der Atemströmungskurve als Triggerkriterium benutzt wird.

6. Verfahren nach einem der Ansprüche 1 bis 4, dadurch gekennzeichnet, daß an die Atemströmungskurve jeweils zwischen ihren Nulldurchgängen eine mathematische Funktion angepaßt wird, und eine signifikante Abweichung der Atemströmungskurve von der mathematischen Funktion als Triggerkriterium benutzt wird.

7. Verfahren nach einem der Ansprüche 1 bis 4, dadurch gekennzeichnet, daß durch Anpassung einer mathematischen Funktion an die Atemströmungskurve jeweils zwischen ihren Nulldurchgängen während mehrerer Atemzüge gemittelte Parameter für den Abfall der Atemströmungskurve während der Inspirations- und der Exspirationsphase gewonnen werden, mit diesen Parametern und dem jeweils gemessenen Extremwert der Atemströmungskurve der voraussichtliche Verlauf der Atemströmungskurve vorausberechnet wird und eine signifikante Abweichung des gemessenen von dem vorausberechneten Verlauf der Atemströmungskurve jeweils zwischen ihren Nulldurchgängen als Triggerkriterium benutzt wird.

8. Verfahren nach einem der Ansprüche 1 bis 4, dadurch gekennzeichnet, daß ein Rechnermodell der Patientenlunge und des Beatmungsgerätes synchron zur Atmung des Patienten läuft, wobei die Modellparameter laufend an die gemessene Atemströmungskurve angepaßt werden und die in dem Rechnermodell auftretende Zunahme der Steilheit der berechneten Atemströmungskurve jeweils zwischen ihren Nulldurchgängen als Triggerkriterium benutzt wird.

9. Verfahren nach Anspruch 8, dadurch gekennzeichnet, daß eine signifikante Abweichung des gemessenen von dem mittels des Rechnermodells vorausberechneten Verlaufs der Atemströmungskurve jeweils zwischen ihren Nulldurchgängen als Triggerkriterium benutzt wird.

## Claims

1. Method for recognizing the breathing phases of a patient for the control of a respiration apparatus, wherein the time course of a signal, which represents the respiratory-flow curve and is emitted by a signalling device, is determined and a trigger criterium is acquired from this course for the reversal of the respiration apparatus from the expiratory phase to the inspiratory phase and/or from the inspiratory phase to the expiratory phase, characterised in that a significant increase in the steepness of the respiratory-flow curve at any one time between its zero crossings is used as the trigger criterium.

2. Method according to claim 1, characterised in that only that significant increase in the steepness of the respiratory-flow curve at any one time between its zero crossings, that occurs after the extreme value at any one time of the respiratory-flow curve has been exceeded, is used as the trigger criterium.

3. Method according to claim 1 or 2, characterised in that only that significant increase in the steepness of the respiratory-flow curve that occurs after a time-delay has elapsed, which time-delay begins with every zero crossing of the respiratory-flow curve, is used as the trigger criterium.

4. Method according to claim 3, characterised in that a value of between 50 ms and 800 ms is chosen for the time-delay.

5. Method according to one of the claims 1 to 4, characterised in that the signal representing the respiratory-flow curve is low-pass filtered, and subsequently the double time differential is formed, and a change in the signal thus acquired, which change represents a significant increase in the steepness of the respiratory-flow curve at any one time between the zero crossings of the respiratory-flow curve is used as the trigger criterium.

6. Method according to one of the claims 1 to 4, characterised in that a mathematical function is matched to the respiratory-flow curve at any one time between its zero crossings, and a significant deviation of the respiratory-flow curve from the mathematical function is used as the trigger criterium.

7. Method according to one of the claims 1 to 4, characterised in that by matching a mathematical function to the respiratory-flow curve at any one time between its zero crossings, parameters averaged for several breaths are acquired for the drop in the respiratory-flow curve during the inspiratory and the expiratory phases, the estimated course of the respiratory-flow curve is predicted using these parameters and the measured extreme value of the respiratory-flow curve at any one time, and a significant deviation of the measured course from the predicted course of the respiratory-flow curve at any one time between its zero crossings is used as the trigger criterium.

8. Method according to one of the claims 1 to 4, characterised in that a computer model of the patient's lungs and the respiration apparatus runs synchronously with the breathing of the patient, wherein the parameters of the model are continuously matched to the measured respiratory-flow curve and the increase in the steepness of the calculated respiratory-flow curve at any one time between its zero crossings, which increase occurs in the computer model, is used as the trigger criterium.

9. Method according to claim 8, characterised in that a significant deviation of the measured course from the course of the respiratory-flow curve predicted by means of the computer model that is at any time between its zero crossings is used as the trigger criterium.

## Revendications

1. Procédé de détection des phases respiratoires d'un patient pour la commande d'un appareil d'assistance respiratoire, la variation dans le temps d'un signal émis par un dispositif de signalisation et représentatif de la courbe de débit respiratoire étant indiquée et un critère de déclenchement pour l'inversion du fonctionnement de l'appareil d'assistance respiratoire étant obtenu à partir de cette variation, pour le faire passer de la phase d'expiration à la phase d'inspiration et/ou de la phase d'inspiration à la phase d'expiration, caractérisé en ce qu'une augmentation significative de la raideur de pente de la courbe de débit respiratoire entre chacun de ses passages à zéro est utilisée comme critère de déclenchement.

2. Procédé selon la revendication 1, caractérisé en ce que seule l'augmentation significative de la raideur de pente de la courbe de débit respiratoire, entre chacun de ses passages à zéro, apparaissant après le dépassement de la valeur extrème respective de la courbe de débit respiratoire, est utilisée comme critère de déclenchement.

3. Procédé selon la revendication 1 ou 2, caractérisé en ce que seule l'augmentation significative de la raideur de pente de la courbe de débit respiratoire, apparaissant après que se soit écoulé un temps de retard débutant à chaque passage à zéro de la courbe de débit respiratoire, est utilisée comme critère de déclenchement.

4. Procédé selon la revendication 3, caractérisé en ce qu'une valeur située entre 50 ms et 800 ms est sélectionnée pour le temps de retard.

5. Procédé selon l'une des revendications 1 à 4, caractérisé en ce que le signal représentatif de la courbe de débit respiratoire est filtré à travers un passe-bas et la dérivée seconde par rapport au temps est ensuite calculée, et une variation, représentative d'une augmentation significative de la raideur de pente de la courbe de débit respiratoire, du signal ainsi obtenu, entre chacun des passages à zéro de la courbe de débit respiratoire, est utilisée code critère de déclenchement.

6. Procédé selon l'une des revendications 1 à 4, caractérisé en ce qu'une fonction mathématique est adaptée à la courbe de débit respiratoire, entre chacun de ses passages à zéro, et une variation significative de la courbe de débit respiratoire par rapport à la fonction mathématique est utilisée comme critère de déclenchement.

7. Procédé selon l'une des revendications 1 à 4, caractérisé en ce que, par l'adaptation d'une fonction mathématique à la courbe de débit respiratoire, entre chacun de ses passages à zéro, pendant plusieurs mouvements respiratoires, des paramètres pondérés sont calculés pour la chute de la courbe de débit respiratoire au cours de la phase d'inspiration et de la phase d'expiration, le tracé prévisible de la courbe de débit respiratoire est calculé d'avance à l'aide de ces paramètres et de la valeur extrème, respectivement mesurée, de la courbe de débit respiratoire, et une variation significative entre le tracé mesuré et le tracé calculé d'avance de la courbe de débit respiratoire, entre chacun de ses passages à zéro, sert de critère de déclenchement.

8. Procédé selon l'une des revendications 1 à 4, caractérisé en ce qu'un modèle mathématique du poumon du patient et de l'appareil d'assistance respiratoire, fonctionne de façon synchrone avec la respiration du patient, les paramètres du modèle étant continuellement adaptés à la courbe de débit respiratoire, l'augmentation de la raideur de pente de la courbe de débit respiratoire mesurée, entre chacun de ses passages à zéro, apparaissant dans le modèle mathématique, étant utilisée comme critère de déclenchement.

9. Procédé selon la revendication 8, caractérisé en ce qu'une variation significative du tracé mesuré de la courbe de débit respiratoire par rapport au tracé calculé d'avance par le modèle mathématique, entre chacun de ses passages à zéro, est utilisée comme critère de déclenchement.
